# EUROPÄISCHE PATENTSCHRIFT

(11) **EP 0 906 567 B1**
(45) Veröffentlichungstag und Bekanntmachung des Hinweises auf die Patenterteilung: **14.03.2001**
(21) Anmeldenummer: 97927108.7
(22) Anmeldetag: 02.06.1997
(51) Int. Cl.: G01N 33/487, G01N 33/68

(54) **VERFAHREN ZUR BESEITIGUNG VON HÄMOGLOBIN-STÖRUNGEN BEI DER BESTIMMUNG VON ALBUMIN**
PROCESS TO ELIMINATE HAEMOGLOBIN ERRORS DURING THE DETERMINATION OF ALBUMIN
PROCEDE POUR SUPPRIMER LES ERREURS DUES A L'HEMOGLOBINE LORS DE LA DETERMINATION DE LA PRESENCE D'ALBUMINE

(30) Priorität: 31.05.1996 DE 19622091; 28.05.1997 US 47997 P
(43) Veröffentlichungstag der Anmeldung: 07.04.1999
(73) Patentinhaber: Roche Diagnostics GmbH, 68305 Mannheim (DE)
(72) Erfinder: WEISHEIT, Ralph, D-82362 Weilheim (DE); MASTERS, Barbara, Boehringer Mannheim Corporation, Indianapolis, IN 46250-0457 (US)
(74) Vertreter: Weiss, Wolfgang, Dipl.-Chem. Dr.
(86) Internationale Anmeldenummer: EP9702862
(87) Internationale Veröffentlichungsnummer: WO9745728

(56) Entgegenhaltungen:
- EP-A- 0 268 025
- EP-A- 0 695 805
- US-A- 5 284 777
- PATENT ABSTRACTS OF JAPAN vol. 012, no. 441 (P-789), 21.November 1988 & JP 63 169564 A (SHIMADZU CORP), 13.Juli 1988,

## Beschreibung

Die vorliegende Erfindung betrifft ein Verfahren zur Bestimmung von Albumin in einer freies Hämoglobin enthaltenden Probe durch optische Messung.

Bekanntermaßen wird die nichtimmunologische Bestimmung von Albumin in Serum und Plasma (z.B. mittels Bromcresolgrün-Methode) durch Hämoglobin (Hb) und Hämoglobin-analoge Verbindungen derart gestört, daß bei Anwesenheit dieser Störsubstanzen falsch erhöhte Albuminwerte erhalten werden. Die Ursache hierfür liegt in einer Reaktion von Hämoglobin mit dem Farbstoff, wodurch Hb quantitativ als Albumin erfaßt wird (Doumas et al, Clin. Chim. Acta 31, 87-96 (1971)).

Im Patent EP 0 268 025 B1 wird darauf verwiesen, daß die Hb-Störung des Albumin-Tests nicht durch eine konventionelle 2Punkt-Messung beseitigt werden kann. Es wird jedoch vorgeschlagen, aus dem Zusammenhang zwischen Hämolysegrad und Meßfehler einen Korrekturfaktor zu ermitteln und mit dessen Hilfe (im Anschluß an eine separate Bestimmung des Hämolysegrades) das für eine bestimmte Probe erhaltene fehlerhafte Albuminergebnis rechnerisch zu korrigieren.

Jay und Provasek (Clin. Chem. 39/9, 1804-1810 (1993)) beschrieben, daß in ihrem Labor bei jeder erkennbar hämolytischen Probe der Hb-Gehalt dieser Probe separat bestimmt und über ein Computerprogramm das (verfälschte) Ergebnis der Analytbestimmung rechnerisch korrigiert wird. Die Höhe dieser Korrektur wird anschließend auf dem Analysenbefund angegeben.

Eine weitere Möglichkeit zur Korrektur der Hb-Störung für Albumin beschreibt die Offenlegungsschrift DE 4427492 A1. Hier entfällt eine separate Bestimmung des Hämolysegrades, da dieser aus einer der eigentlichen Hauptreaktion vorgeschalteten Vorreaktion abgeleitet werden kann. Nach dem sogenannten Rateₚₗᵤₛ-Verfahren wird das in der Hauptreaktion erhaltene Meßergebnis unter Ausnutzung eines gefundenen Zusammenhanges zwischen Hämolysegrad und Störbeitrag rechnerisch korrigiert.

In den bisher beschriebenen Methoden zur Beseitigung der Hb-Störung bei Albumin-Bestimmungen ist generell eine rechnerische Korrektur des Analysenergebnisses um einen dem Hb-Gehalt entsprechenden Betrag erforderlich. Dabei wird der Hb-Gehalt bzw. Hämolysegrad entweder durch separate Messung (z.B. durch Multiwellenlängenanalyse oder eine unabhängige Methode) ermittelt oder durch eine Vorreaktion abgeleitet, wobei immer ein 2-Reagenzien-Test erforderlich ist.

Die Bestimmung von Albumin erfolgt derzeit jedoch häufig als 1-Punkt-Messung mittels eines Monoreagenzes, wobei die Farbreaktion durch Vermischen von Probe und Reagenz gestartet wird (z.B. Albumin-Reagenz der Fa. Boehringer Mannheim GmbH). Der Vorteil einer solchen Bestimmung ist neben der einfachen Handhabung auch ein niedriger Preis. Aufwendig ist jedoch die Beseitigung der Hb-Störung: Entweder Monoreagenz und separate Ermittlung des Hb-Gehaltes oder ein 2-Reagenzien-Test und Vermeidung einer separaten Ermittlung des Hb-Gehaltes. In jedem Fall muß das erhaltene Analysenergebnis anschließend rechnerisch korrigiert werden.

Darüber hinaus wird bei der Bestimmung von Hämoglobin enthaltenden Serum- oder Plasmaproben die Hauptreaktion (Albumin mit dem Testreagenz) durch eine Störreaktion (Hämoglobin mit dem Testreagenz) und einer im Zusammenhang mit der Störreaktion verbundenen Abnahme des Absorptionssignals von Hämoglobin selbst überlagert. Zusätzlich unterscheiden sich die Absorptionsspektren sowohl der Hauptreaktion als auch der Störreaktion in der Abhängigkeit von den jeweils verwendeten Albumintestreagenzien.

Überraschend wurde gefunden, daß der Einfluß der Störreaktion auf das Ergebnis der Hauptreaktion abhängig ist von der Kombination der verwendeten Meßwellenlängen und darüber hinaus auch von der Reaktionszeit.

Die Beseitigung der Hämoglobinstörung bei der Bestimmung von Albumin ist damit sehr komplex, da es sich hierbei nicht um ein Störsignal im üblichen Sinn handelt, das über eine einfache bichromatische Messung bzw. über eine einfache Messung des Probenleerwerts beseitigt werden kann. Vielmehr reagiert die Störsubstanz, d. h. natives, vernetztes, polymerisiertes oder rekombinant hergestelltes Hämoglobin, selbst direkt mit dem Reagenz. Es muß daher nach Meßwellenkombinationen gesucht werden, bei denen diese Störreaktion eindeutig von der eigentlichen Hauptreaktion unterscheidbar ist.

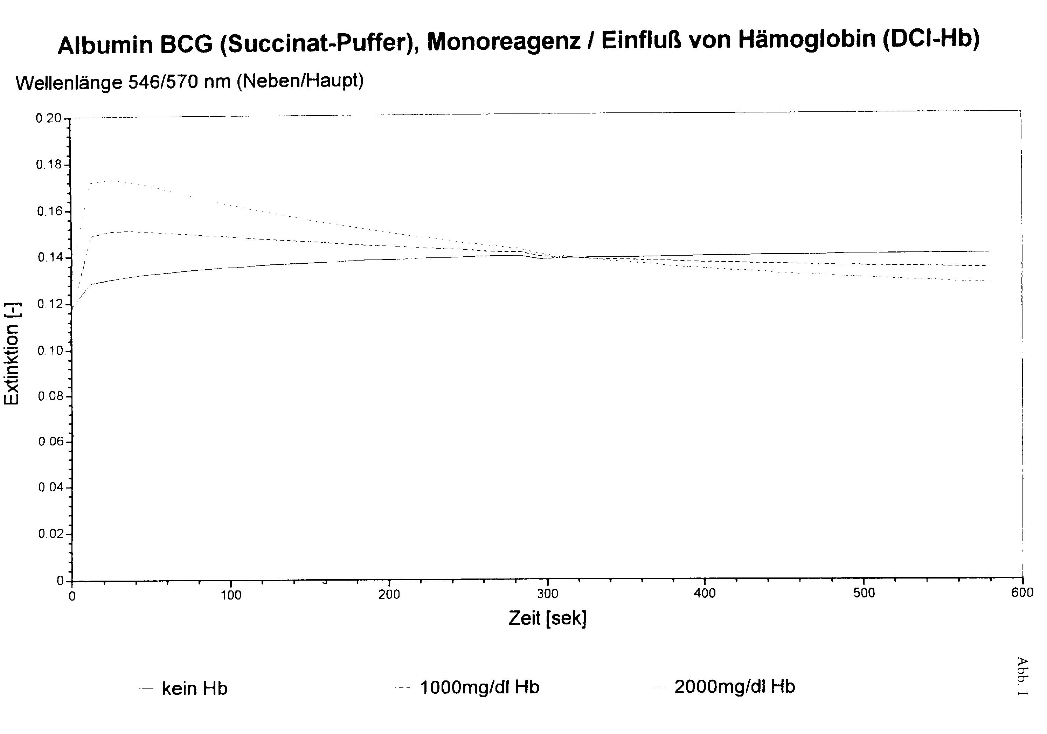

Gelöst wurde diese Aufgabe durch Veränderung der Kombination von Meßwellenlängen, d. h. Haupt- und der Nebenmeßwellenlänge, bei einer bichromatischen Messung. Überraschenderweise wurde gefunden, daß bei veränderten Kombinationen von Wellenlängen sich ein durch freies Hämoglobin in der Probe hervorgerufener Meßfehler weitgehend unterdrücken läßt (siehe Abb. 1, 3, 4, 5, 7, 8, 10 und 11). Bei diesen Wellenlängenkombinationen, die im allgemeinen nicht im Absorptionsmaximum der Testreagenzien liegen, ändert sich das Meßsignal einer hämoglobinhaltigen Probe zeitabhängig, während das Meßsignal einer hämoglobin-freien Probe im wesentlichen konstant bleibt.

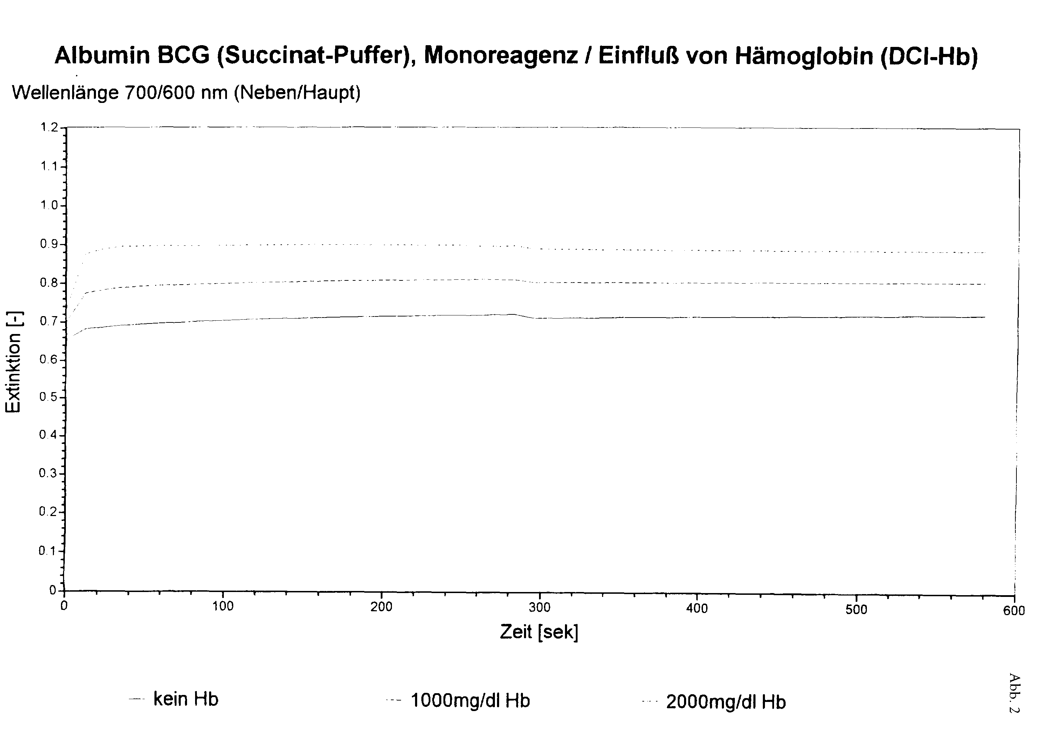

Dieser Effekt wird jedoch nicht beobachtet bei der üblicherweise zur Bestimmung von Albumin verwendeten Wellenlängenkombination, d.h. einer Hauptwellenlänge von 600 nm und einer Nebenwellenlänge von 700 nm. Hier findet man einen hohen Meßfehler bei einer Hämoglobin-haltigen Probe gegenüber einer Hämoglobin-freien Probe (vgl. Abb. 2, 6 und 9).

Ein Gegenstand der vorliegenden Erfindung ist somit ein Verfahren zur Bestimmung von Albumin in einer freies Hämoglobin enthaltenden Probe durch optische Messung bei mindestens zwei Wellenlängen, dadurch gekennzeichnet, daß
(a) bei einer ersten Meßwellenlänge ein zur Bestimmung ausreichend hohes Meßsignal für Albumin vorliegt,
(b) bei einer zweiten Meßwellenlänge (i) kein Meßsignal für Albumin oder (ii) eine im Vergleich zum Meßsignal bei der ersten Meßwellenlänge geringeres Meßsignal für Albumin vorliegt und
(c) bei der ersten und bei der zweiten Meßwellenlänge ein vergleichbar hohes Störsignal vorliegt, das durch eine Reaktion von Hämoglobin mit dem zur Bestimmung von Albumin verwendeten Testreagenz erzeugt wird und das sich zeitabhängig ändert.

Für das erfindungsgemäße Verfahren wird eine Meßwellenlängenkombination verwendet, wobei bei einer ersten Meßwellenlänge ein zur Bestimmung ausreichend hohes Meßsignal für Albumin vorliegt. Dabei ist anzumerken, daß die erste Meßwellenlänge nicht im Absorptionsmaximum der verwendeten Testreagenzien liegen muß, sondern vorzugsweise sogar außerhalb des Absorptionsmaximums liegt. Bei der zweiten Meßwellenlänge liegt ein Meßsignal für Albumin vor, das deutlich geringer als das Signal bei der ersten Meßwellenlänge ist. Vorzugsweise ist das Meßsignal bei der ersten Meßwellenlänge mindestens 20 mE und besonders bevorzugt mindestens 50 mE höher als bei der zweiten Meßwellenlänge.

Weiterhin soll bei der ersten und bei der zweiten Meßwellenlänge ein vergleichbar hohes Störsignal vorliegen, welches sich zeitabhängig ändert. Vorzugsweise wird daher bei einer Einpunktmessung der Meßpunkt so gewählt, daß das durch die Reaktion von Hämoglobin mit dem Testreagenz verursachte Störsignal bei der ersten und bei der zweiten Meßwellenlänge gleich oder annähernd gleich groß ist.

Bei einer Mehrpunktmessung, z.B. einer Zweipunktmessung (2-Reagenzientest mit Messung des Probenleerwerts und des Analytwerts) werden die Meßpunkte vorzugsweise so gewählt, daß unter Berücksichtigung des Verdünnungsfaktors aus den Pipettiervolumina der Einzelreagenzien das Störsignal bei den Meßzeitpunkten, z.B. beim ersten und beim zweiten Meßzeitpunkt, gleich oder annähernd gleich groß ist. Die Mehrpunktmessung wird vorzugsweise als Endpunktmessung durchgeführt, obwohl kinetische Messungen auch möglich sind.

Die Bestimmung des Albumingehalts nach dem erfindungsgemäßen Verfahren erfolgt durch optische Messung über eine Farbstoffreaktion. Vorzugsweise erfolgt die Bestimmung des Albumingehalts nach der Bromcresolgrün- oder Bromcresolpurpur-Methode.

Durch Untersuchungen konnten bisher für die Bestimmung von Albumin mit der Bromcresolgrün-Methode und einem Succinatpuffer drei bevorzugte Kombinationen von Meßwellenlängen ermittelt werden. Bei einer ersten bevorzugten Ausführungsform führt man die Bestimmung bei einer ersten Meßwellenlänge von 560 bis 600 nm, insbesondere von 560 bis 580 nm, besonders bevorzugt von 570 ± 5 nm und am meisten bevorzugt von ca. 570 nm und bei einer zweiten Meßwellenlänge von 540 bis 552 nm, besonders bevorzugt von 546 ± 5 nm und am meisten bevorzugt von ca. 546 nm durch.

In einer zweiten bevorzugten Ausführungsform der vorliegenden Erfindung wird eine erste Meßwellenlänge von 640 bis 680 nm, besonders bevorzugt von 660 ± 5 nm und am meisten bevorzugt von ca. 660 nm und eine zweite Meßwellenlänge von 470 bis 490 nm, besonders bevorzugt von 480 ± 5 nm und am meisten bevorzugt von ca. 480 nm verwendet.

In einer dritten bevorzugten Ausführungsform der vorliegenden Erfindung wird eine erste Meßwellenlänge von 620 bis 640 nm, besonders bevorzugt von 630 ± 5 nm und am meisten bevorzugt von 630 nm und eine zweite Meßwellenlänge von 590 bis 610 nm, besonders bevorzugt von 600 ± 5 nm und am meisten bevorzugt von ca. 600 nm verwendet.

Für die Bestimmung von Albumin mit der Bromcresolgrün-Methode und einem Citrat-Puffer wird bevorzugt eine erste Meßwellenlänge von 560 bis 580 nm, besonders bevorzugt von 570 ± 5 nm und am meisten bevorzugt von ca. 570 nm und eine zweite Meßwellenlänge von 490 bis 520 nm, besonders bevorzugt von 505 ± 5 nm und am meisten bevorzugt von ca. 505 nm verwendet.

Bei der Verwendung anderer Testreagenzien oder/und anderer Puffersubstanzen kann der Fachmann durch einfache Versuche, z.B. die Analysen von Diodenarray-Spektren und zeitabhängige Absorptionsmessungen, weitere geeignete Wellenlängenkombinationen identifizieren.

Mit Hilfe dieser Wellenlängenkombinationen wird überraschenderweise bei Messung einer Hämoglobin enthaltenden Probe ein zumindest weitgehend unverfälschter Wert für die Albuminkonzentration in der Probe erhalten, der anschließend nicht mehr rechnerisch korrigiert werden muß. Durch das erfindungsgemäße Verfahren kann eine Wiederfindung von Albumin im Bereich von 100 ± 10% und vorzugsweise von 100 ± 5% erreicht werden, insbesondere bei Albuminwerten im unteren medizinischen Entscheidungsbereich von ca. 3,5 g/dl.

Die optimale Reaktionszeit für das erfindungsgemäße Verfahren kann durch einfache Vorversuche aus einem Absorptions/Zeit-Diagramm, in dem der Reaktionsverlauf dargestellt ist, abgeleitet werden und liegt im allgemeinen im Bereich von 0,2 - 10 min. Für die Bestimmung von Albumin mit der Bromcresolgrün-Methode in einem Succinat-Puffer wurde bei einer Probe, die Hämoglobin aus Hämolyse enthält, festgestellt, daß bei Messung bei der Wellenlängenkomination (a) das Meßsignal innerhalb einer Reaktionszeit von 1 - 10 min im wesentlichen konstant bleibt, so daß die Messung zu beliebigen Zeitpunkten innerhalb dieses Bereichs oder gegebenenfalls auch danach erfolgen kann.

Bei einer Probe, die als Blutersatzmittel ein vernetztes Hämoglobin, z.B. Diaspirin-crosslinked (DCL) Hämoglobin enthält, führt man eine Einpunktmessung mit einem Monoreagenz bei der Wellenlängenkombination (a) vorzugsweise nach einer Reaktionszeit von 3 - 10 min, besonders bevorzugt von 5 - 7 min und am meisten bevorzugt von ca. 6 min durch. Bei der Wellenlängenkombination (b) führt man hingegen die Messung vorzugsweise nach einer Reaktionszeit von 1 - 4 min, besonders bevorzugt von 2 - 3 min und am meisten bevorzugt von ca. 2,5 min nach dem Reaktionsstart durch.

Bei einer Probe, die als Blutersatzmittel rekombinant hergestelltes Hämoglobin enthält, wird eine Einpunktmessung mit einem Monoreagenz bei der Wellenlängenkombination (a) vorzugsweise nach einer Reaktionszeit von 1 - 3 min, besonders bevorzugt nach 1 - 2 min und am meisten bevorzugt nach ca. 1,5 min durchgeführt. Bei der Wellenlängenkombination (b) erfolgt eine Einpunktmessung vorzugsweise nach einer Reaktionszeit von 1 - 4 min.

Für die Bestimmung von Albumin mit der Bromcresolgrün-Methode in einem Citrat-Puffer wird bei einer Probe, die DCL-Hämoglobin enthält, die Analytwert-Messung bei einem Monoreagenz vorzugsweise nach 20 bis 90 sec und besonders bevorzugt nach 30 bis 60 sec und bei einem 2-Reagenzientest vorzugsweise nach 0,2 bis 5 min nach Zugabe von Reagenz 2 (Startreagenz) durchgeführt.

Auch für die Bestimmung von Albumin mit der Bromcresolpurpur-Methode konnten zwei bevorzugte Kombinationen von Meßwellenlängen ermittelt werden, bei der eine Störung durch freies Hämoglobin in der Probe weitgehend beseitigt werden kann. Bei einer ersten bevorzugten Ausführungsform führt man die Bestimmung bei einer ersten Meßwellenlänge von 560 bis 580 nm, besonders bevorzugt von 570 ± 5 nm und am meisten bevorzugt von ca. 570 nm und bei einer zweiten Meßwellenlänge von 490 bis 520 nm, besonders bevorzugt 505 ± 5 nm und am meisten bevorzugt von ca. 505 nm durch.

Bei einer zweiten bevorzugten Ausführungsform der vorliegenden Erfindung wird eine erste Meßwellenlänge von 560 bis 580 nm, besonders bevorzugt von 570 ± 5 nm und am meisten bevorzugt von ca. 570 nm und eine zweite Meßwellenlänge von 540 bis 552 nm, besonders bevorzugt von 546 ± 5 nm und am meisten bevorzugt von ca. 546 nm verwendet.

Bei einer Probe, die als Blutersatzmittel ein vernetztes Hämoglobin, z.B. DCL-Hämoglobin enthält, wird die Analytwert-Messung bei einem 2-Reagenzientest vorzugsweise 0,2 bis 8 min, besonders bevorzugt ca. 5 min nach Zugabe von Reagenz 2 (Startreagenz) durchgeführt. Die Probenleerwert-Messung wird bei der ersten Wellenlängenkombination (570/546 nm) vorzugsweise 1 bis 4 min, besonders bevorzugt ca. 2,5 min nach Zugabe von Reagenz 1 zur Probe durchgeführt. Bei der zweiten Wellenlängenkombination erfolgt die Probenleerwertmessung vorzugsweise 0,2 bis 4 min, besonders bevorzugt 0,2 bis 1 min und am meisten bevorzugt ca. 0,5 min nach Zugabe von Reagenz 1 zur Probe.

Entscheidend für das erfindungsgemäße Verfahren ist jedoch die neuartige Kombination von Haupt- und Meßwellenlänge. Die Messung bei unterschiedlichen optimalen Meßzeitpunkten für verschiedene Hb-Arten kann jedoch zu einer weiteren Verringerung des Meßfehlers verwendet werden.

Auf diese Weise ist es erstmals möglich, ohne zusätzliche Ermittlung des Hämoglobingehaltes oder Hämolysegrads und ohne anschließende rechnerische Korrektur eines durch Hämoglobin verfälschten Analysenergebnisses den korrekten Albumingehalt in nur einem Meßschritt, d.h. als 1-Punkt-Messung zu bestimmen. Ein weiterer entscheidender Vorteil ist es, daß die Bestimmung korrekter Albuminwerte auch mit einem Monoreagenz möglich ist. Dies ist mit erheblichen Handhabungs- und Preisvorteilen verbunden.

Ein 2-Reagenzientest kann weiterhin verwendet werden, ist jedoch nicht mehr zwingend erforderlich. Aber auch für einen 2-Reagenzientest ergibt sich durch die vorliegende Erfindung der Vorteil, daß die Ermittlung des Hämolysegrades mit anschließender rechnerischer Korrektur der durch Hämoglobin verfälschten Meßwerte für Albumin vermieden werden kann.

Als Hämoglobin-enthaltende Proben werden üblicherweise Serum- oder Plasmaproben verwendet. Dabei ist anzumerken, daß die Bezeichnung "freies Hämoglobin" im Sinne der vorliegenden Erfindung sich sowohl auf hämolytische Proben als auch auf Proben bezieht, denen eine Hämoglobin-analoge Verbindung als Blutersatzmittel, z.B. ein modifiziertes, polymerisiertes oder/und quervernetztes Derivat von Human- oder Rinderhämoglobin, z.B. Diaspirin-crosslinked (DCL)-Hämoglobin, oder auch ein rekombinant hergestelltes Hämoglobin zugesetzt worden ist.

Bevorzugt ist es außerdem, daß das erfindungsgemäße Verfahren an einem Analyseautomaten durchgeführt wird. Ein Beispiel für einen geeigneten Analyseautomaten ist das Boehringer Mannheim/Hitachi 717-Gerät.

Weiterhin soll die vorliegende Erfindung durch Abbildungen und Beispiele erläutert werden.

Es zeigen:
- Abb. 1: den zeitabhängigen Verlauf des Meßsignals bei der Bestimmung von Albumin mit einem Monoreagenz bei einer Meßwellenlängenkombination 546/570 nm (Succinatpuffer) in Anwesenheit von vernetztem Hämoglobin (DCL-Hb) gemäß vorliegender Erfindung,
- Abb. 2: den zeitabhängigen Verlauf des Meßsignals bei der Bestimmung von Albumin mit einem Monoreagenz bei einer Meßwellenlängenkombination 700/600 nm (Succinatpuffer) in Anwesenheit von vernetztem Hämoglobin (DCL-Hb) gemäß dem Stand der Technik,
- Abb. 3: den zeitabhängigen Verlauf des Meßsignals bei der Bestimmung von Albumin mit einem Monoreagenz bei einer Meßwellenlängenkombination 546/570 nm (Succinatpuffer) in Anwesenheit von rekombinant hergestelltem Hämoglobin gemäß vorliegender Erfindung und
- Abb. 4: den zeitabhängigen Verlauf des Meßsignals bei der Bestimmung von Albumin mit einem Monoreagenz bei einer Meßwellenlängenkombination 546/570 nm (Succinatpuffer) in Anwesenheit von Hämoglobin aus Hämolyse gemäß vorliegender Erfindung,
- Abb. 5: den zeitabhängigen Verlauf des Meßsignals bei der Bestimmung von Albumin mit einem Monoreagenz bei einer Meßwellenlängenkomination 480/660 nm (Succinatpuffer) in Anwesenheit von DCL-Hämoglobin gemäß vorliegender Erfindung,
- Abb. 6: den zeitabhängigen Verlauf des Meßsignals bei der Bestimmung von Albumin mit einem Monoreagenz bei einer Meßwellenlängenkombination 700/600 nm (Citratpuffer) in Anwesenheit von DCL-Hämoglobin gemäß dem Stand der Technik,
- Abb. 7: den zeitabhängigen Verlauf des Meßsignals bei der Bestimmung von Albumin mit einem Monoreagenz bei einer Meßwellenlängenkombination 505/570 nm (Citrat-puffer) in Anwesenheit von DCL-Hämoglobin gemäß vorliegender Erfindung,
- Abb. 8: den zeitabhängigen Verlauf des Meßsignals bei der Bestimmung von Albumin mit einem Zweireagenzientest bei einer Meßwellenlängenkombination 505/570 nm (Citratpuffer) in Anwesenheit von DCL-Hämoglobin gemäß vorliegender Erfindung,
- Abb. 9: den zeitabhängigen Verlauf des Meßsignals bei der Bestimmung von Albumin nach der Bromcresolpurpur-Methode mit einem Zweireagenzientest bei einer Meßwellenlängenkombination 700/600 nm in Anwesenheit von DCL-Hämoglobin gemäß dem Stand der Technik,
- Abb. 10: den zeitabhängigen Verlauf des Meßsignals bei der Bestimmung von Albumin nach der Bromcresolpurpur-Methode mit einem Zweireagenzientest bei einer Meßwellenlängenkombination 505/570 nm in Anwesenheit von DCL-Hämoglobin gemäß vorliegender Erfindung,
- Abb. 11: den zeitabhängigen Verlauf des Meßsignals bei der Bestimmung von Albumin nach der Bromcresolpurpur-Methode mit einem Zweireagenzientest bei einer Meßwellenlängenkombination 546/570 nm in Anwesenheit von DCL-Hämoglobin gemäß vorliegender Erfindung,
- Abb. 12: ein Diodenarray-Spektrum für die Bestimmung von Albumin mit einem Monoreagenz (Succinatpuffer) und
- Abb. 13: ein Diodenarray-Spektrum für die Bestimmung von Albumin mit einem Monoreagenz (Citratpuffer).

### Beispiel 1

Es wurde eine Bestimmung von Albumin mit einem Monoreagenz nach der Bromcresolgrün-Methode (Doumas et al., Clin. Chim. Acta 31 (1971), 87-96) in einem Succinatpuffer durchgeführt. Als Reagenz wurde verwendet:
Succinatpuffer 75 mmol/l, pH 4,2; 0,15 mmol/l Bromcresolgrün

Die Testdurchführung war wie folgt:

Zu 3 µl Probe wurden 350 µl Reagenz gegeben und dann der zeitabhängige Verlauf des Meßsignals (mE) bestimmt.

Die Bestimmung erfolgte in einem Boehringer Mannheim/Hitachi 717-Analysegerät unter Verwendung der Meßwellenlängenkombinationen 700/600 nm (Stand der Technik) sowie 546/570 nm bzw. 480/660 nm (Erfindung).

Der zeitabhängige Verlauf des Meßsignals für Proben ohne Hämoglobin, mit 1000 mg/dl Hämoglobin und mit 2000 mg/dl Hämoglobin in Form von vernetztem Hämoglobin (DCL-Hb) ist für die Wellenlängenkombination 546/570 nm in Abb. 1 und für die Wellenlängenkombination 700/600 nm in Abb. 2 dargestellt.

Es ist zu erkennen, daß bei der Wellenlängenkombination 546/570 nm mit zunehmender Reaktionsdauer eine Abnahme des Meßsignals bei den hämoglobinhaltigen Proben gefunden wird, während das Meßsignal für die Probe ohne Hämoglobin im wesentlichen konstant bleibt. Die bevorzugte Reaktionsdauer, bei der das Meßsignal dem Signal einer Hb-freien Probe entspricht, ist ca. 5-7 min. Abb. 2 zeigt, daß bei Verwendung der Meßwellenlängenkombination 700/600 nm sowohl die Signale der hämoglobinfreien Probe als auch die Signale der Hämoglobin enthaltenden Proben im wesentlichen konstant bleiben.

In Tabelle 1 ist die prozentuale Wiederfindung des Albumingehalts in Proben mit unterschiedlichem Hämoglobingehalt gezeigt. Bei der Meßwellenlängenkombination 700/600 nm gemäß Stand der Technik (Messung nach einer Reaktionsdauer von 80 s) wurde mit zunehmendem Hämoglobingehalt eine starke Meßwertverfälschung festgestellt. Bei den erfindungsgemäßen Meßwellenlängenkombinationen 546/570 nm (Messung nach einer Reaktionsdauer von 340 s) und 480/660 nm (Messung nach einer Reaktionsdauer von 140 s) wurde hingegen eine vom Hämoglobingehalt der Probe unabhängige Wiederfindung von 100 ± 2 % erreicht.

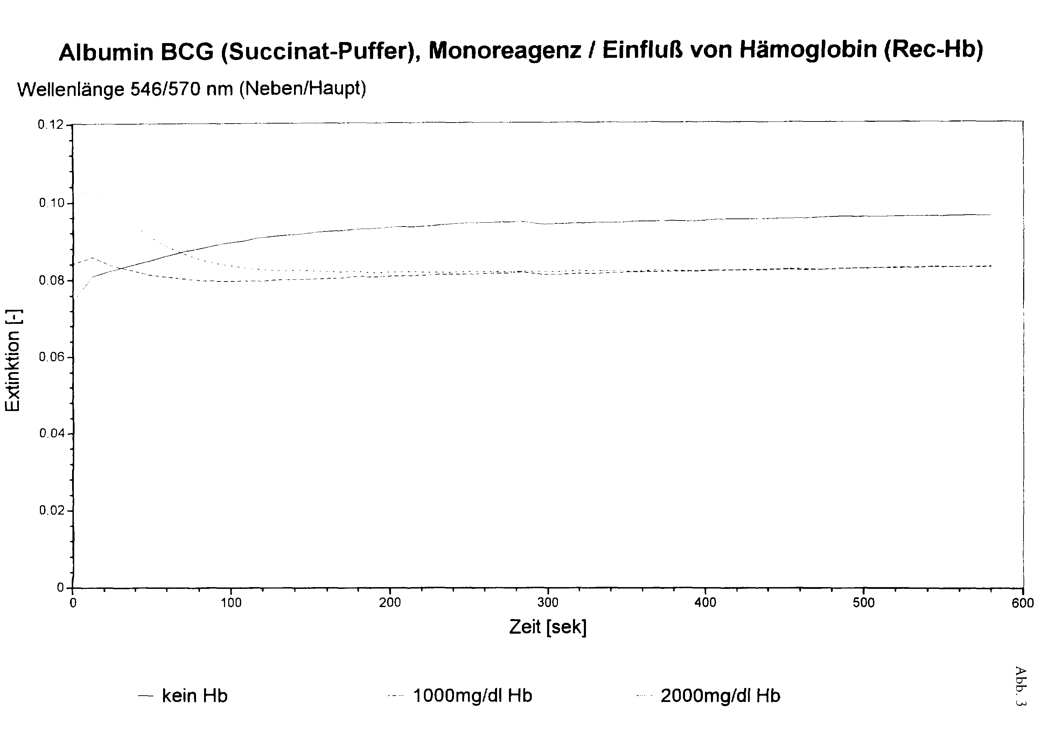

Abb. 3 zeigt den zeitabhängigen Verlauf des Meßsignals für Proben ohne Hb sowie mit 1000 bzw. 2000 mg/dl rekombinant hergestelltem Hämoglobin für die Wellenlängenkombination 546/570 nm. Die bevorzugte Reaktionsdauer ist ca. 1-2 min.

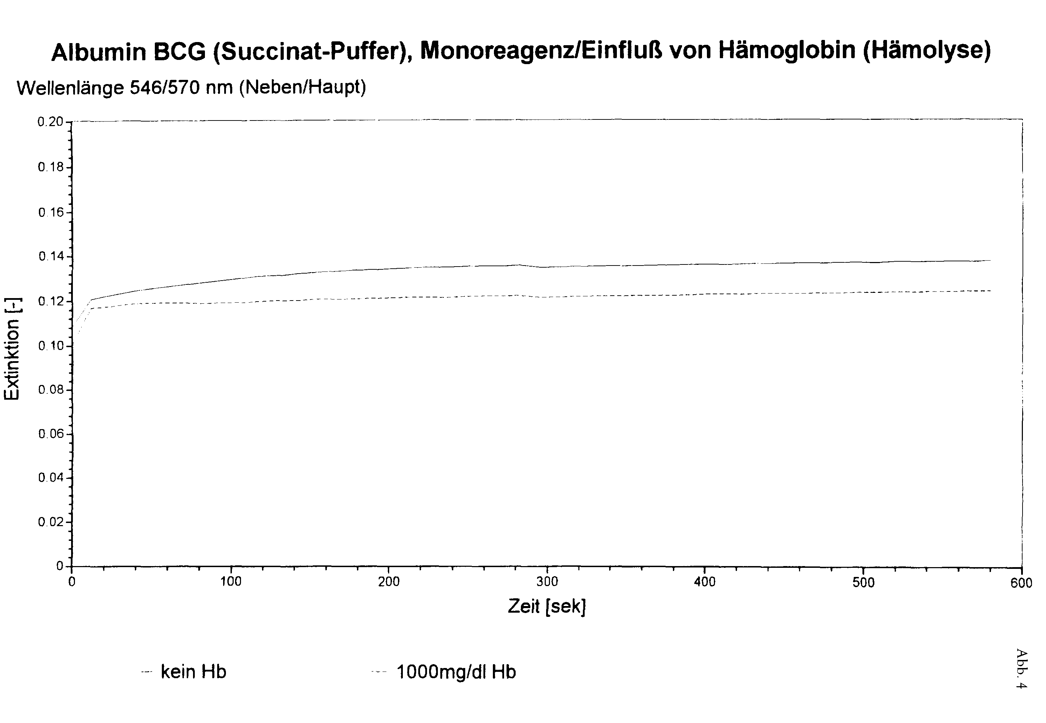

Abb. 4 zeigt den zeitabhängigen Verlauf des Meßsignals für Proben ohne Hb sowie mit 1000 mg/dl Hämoglobin aus Hämolyse.

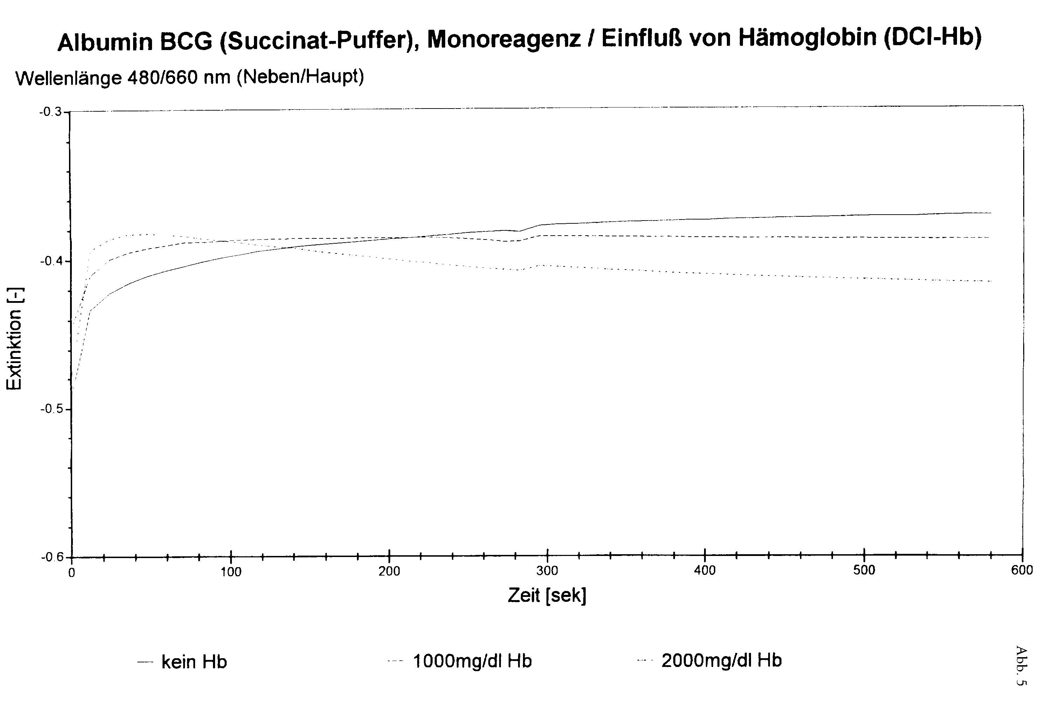

Abb. 5 zeigt den zeitabhängigen Verlauf des Meßsignals für Proben ohne Hb sowie mit 1000 mg/dl bzw. 2000 mg/dl Hb bei der erfindungsgemäßen Meßwellenkombination 480/660 nm. Die bevorzugte Reaktionsdauer, bei der das Meßsignal der DCL-Hb-haltigen Probe dem Signal einer Hb-freien Probe entspricht ist ca. 1 - 3 min.

Bei Proben, die Hämoglobin aus Hämolyse oder rekombinant hergestelltes Hämoglobin enthalten, konnte unter Verwendung der erfindungsgemäßen Wellenlängenkombination 480/660 nm ebenfalls eine gute Entstörung erreicht werden.

### Beispiel 2

Es wurde eine Bestimmung von Albumin mit einem Monoreagenz nach der Bromcresolgrün-Methode in einem Citratpuffer (95 mmol/l, pH 4,1; 0,11 mmol/l Bromcresolgrün; Detergenz) durchgeführt.

Die Testdurchführung war wie in Beispiel 1 beschrieben. Die Bestimmung erfolgte unter Verwendung von Meßwellenlängenkombinationen 700/600 nm (Stand der Technik) und 505/570 nm (Erfindung) sowie einem Meßzeitpunkt von 80 s (Stand der Technik) und 40 - 60 s, bevorzugt 50 s nach Reaktionsstart (Erfindung).

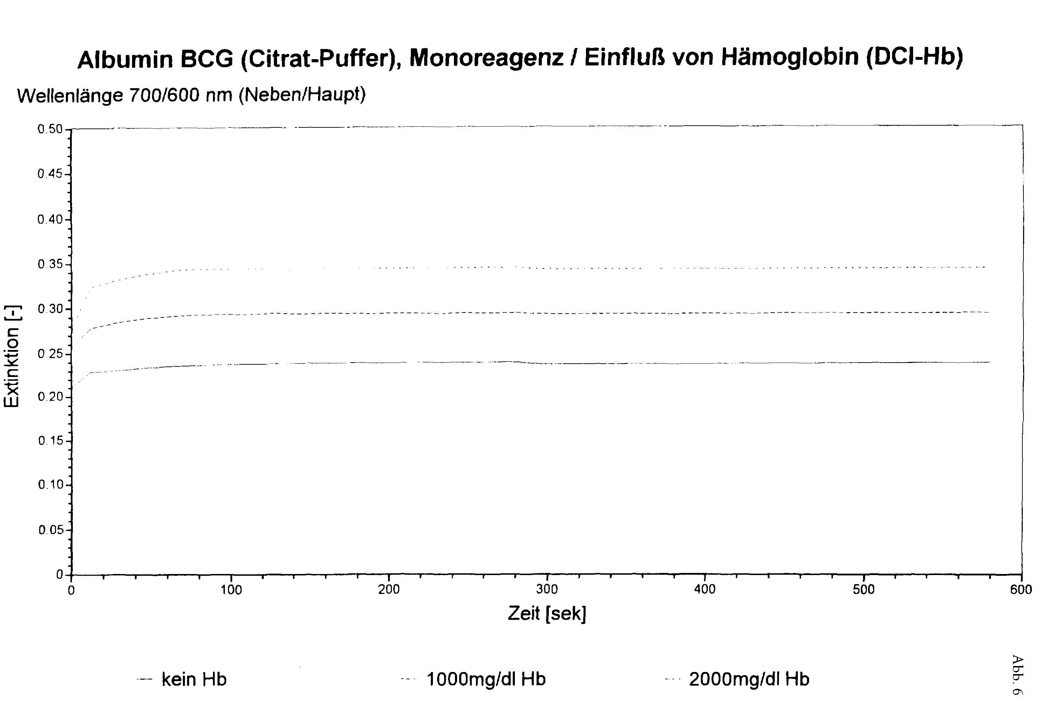

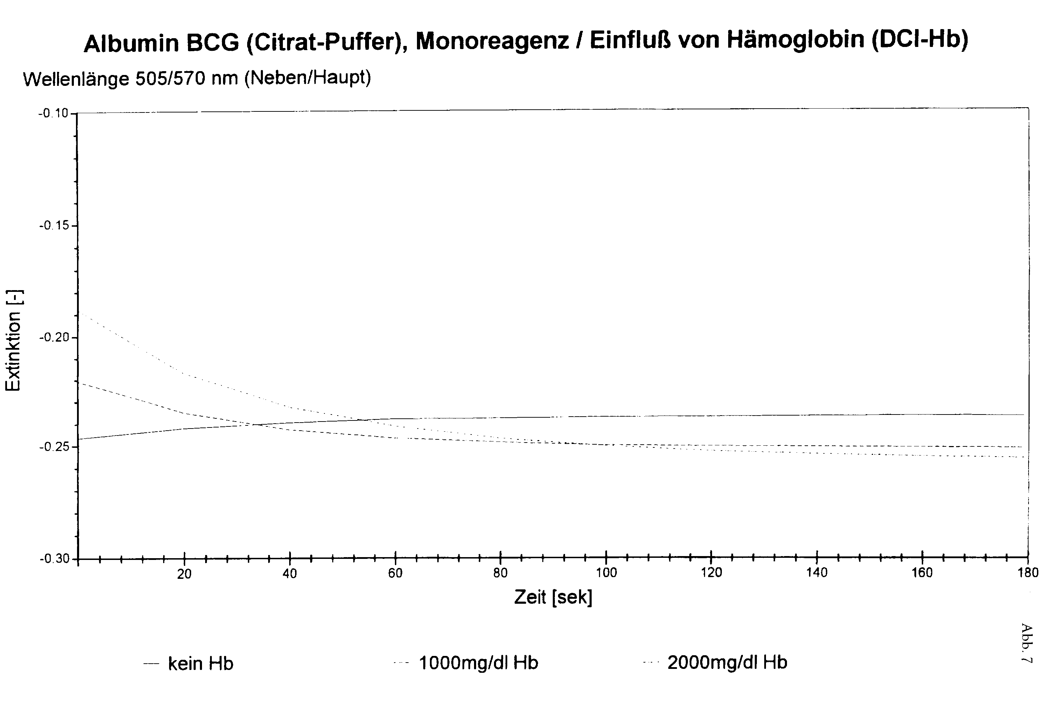

Der zeitabhängige Verlauf des Meßsignals für Proben ohne Hämoglobin, mit 1000 mg/dl Hämoglobin und mit 2000 mg/dl Hämoglobin in Form von DCL-Hämoglobin ist für die Wellenlängenkombination 700/600 nm in Abbildung 6 und für die Wellenlängenkombination 505/570 nm in Abbildung 7 dargestellt.

Es ist zu erkennen, daß bei der erfindungsgemäßen Wellenlängenkombination 505/570 nm mit zunehmender Reaktionsdauer eine Abnahme des Meßsignals bei den hämoglobinhaltigen Proben gefunden wird, während das Meßsignal für die Probe ohne Hämoglobin im wesentlichen konstant bleibt. Bevorzugte Reaktionsdauer, bei der das Meßsignal dem Signal einer Hb-freien Probe entspricht, ist ca. 20 - 90 s.

In Tabelle 2 ist die prozentuale Wiederfindung des Albumingehalts in Proben mit unterschiedlichem Hämoglobingehalt gezeigt. Bei der erfindungsgemäßen Meßwellenlängenkombination 505/570 nm (Messung nach einer Reaktionsdauer von 60 s) wurde eine vom Hämoglobingehalt der Probe unabhängige Wiederfindung von 100 ± 5 % erreicht, während bei der Meßwellenkombination 700/600 nm des Standes der Technik bei zunehmendem Hämoglobingehalt eine starke Meßwertverfälschung festgestellt wurde.

Bei Proben, die Hämoglogin aus Hämolyse oder rekombinant hergestelltes Hämoglobin enthalten, konnte unter Verwendung der erfindungsgemäßen Wellenlängenkombination ebenfalls eine gute Entstörung erreicht werden.

### Beispiel 3

Es wurde eine Bestimmung von Albumin nach der Bromcresolgrün-Methode in einem Citratpuffer mit einem 2-Reagenzientest durchgeführt. Als Reagenzien wurden verwendet:
- Reagenz 1:: Citratpuffer 95 mmol/l, pH 4,1; Detergenz
- Reagenz 2:: Citratpuffer 95 mmol/l, pH 4,1; 0,66 mmol/l Bromcresolgrün; Detergenz

Die Testdurchführung war wie folgt:

Zu 3 µl Probe wurden 250 µl Reagenz 1 gegeben und ca. 4,5 min danach der Probenleerwert E₁ gemessen. Anschließend wurden 50 µl Reagenz 2 zugegeben und 0,5 min danach der Analytmeßwert E₂ gemessen. Außerdem wurde während der gesamten Zeit der zeitabhängige Verlauf des Meßsignals bestimmt. Die Bestimmung erfolgte an einem Boehringer Mannheim/Hitachi 717-Analysengerät unter Verwendung der Meßwellenlängenkombination 505/570 nm (Erfindung). Als Vergleich diente das in Beispiel 2 erwähnte Monoreagenz unter Verwendung der Meßwellenlängenkombination 700/600 nm (Stand der Technik).

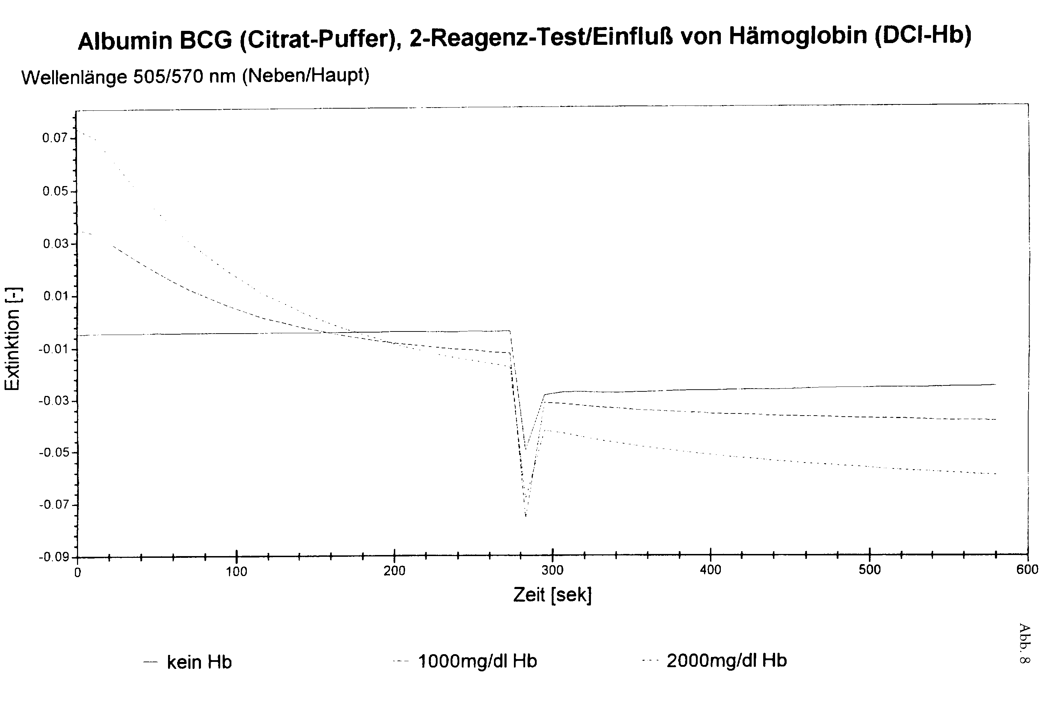

Der zeitabhängige Verlauf des Meßsignals für Proben ohne Hämoglobin, mit 1000 mg/dl Hämoglobin und mit 2000 mg/dl Hämoglobin in Form von DCL-Hämoglobin ist für die erfindungsgemäße Wellenlängenkombination von 505/570 nm in Abb. 8 dargestellt.

Es ist zu erkennen, daß bei einer Wellenlängenkombination von 505/570 nm eine Abnahme des Meßsignals bei den hämoglobinhaltigen Proben gefunden wird. Die bevorzugten Meßzeitpunkte werden so gewählt, daß unter Berücksichtigung des Verdünnungsfaktors aus den Pipettiervolumina das durch Hämoglobin verursachte Störsignal an beiden Meßpunkten gleich hoch ist. Vorzugsweise wird daher E₁ (Probenleerwert) ca. 4,5 min nach Zugabe von Reagenz 1 zur Probe und E₂ (Analytwert) ca. 0,5 min nach Zugabe von Reagenz 2 gemessen.

In Tabelle 3 ist die prozentuale Wiederfindung des Albumingehalts in Proben mit unterschiedlichem Hämoglobingehalt gezeigt. Bei der erfindungsgemäßen Meßwellenlängenkombination 505/570 nm wurde eine vom Hämoglobingehalt der Probe unabhängige Wiederfindung von 100 ± 2 % erreicht, während bei der Meßwellenkombination 700/600 gemäß dem Stand der Technik mit zunehmendem Hämoglobingehalt eine starke Meßwertverfälschung festgestellt wurde.

Bei Proben, die Hämoglogin aus Hämolyse oder rekombinant hergestelltes Hämoglobin enthalten, konnte unter Verwendung der erfindungsgemäßen Wellenlängenkombination ebenfalls eine gute Entstörung erreicht werden.

### Beispiel 4

Es wurde eine Bestimmung von Albumin nach der Bromcresolpurpur-Methode mit einem 2-Reagenzientest und mittels 2-Punkt-messung durchgeführt. Als Reagenzien wurden verwendet:
- Reagenz 1:: Acetat-Puffer 100 mmol/l pH 5,3, Detergenz
- Reagenz 2:: Acetat-Puffer 100 mmol/l pH 5,3, Detergenz, 526 µmol/l Bromcresolpurpur

Die Testdurchführung war wie folgt:

Zu 3 µl Probe wurden 250 µl Reagenz 1 gegeben und ca. 2,5 min danach der Probenleerwert E₁ gemessen. Anschließend wurden 150 µl Reagenz 2 zugegeben und ca. 5 min danach der Analytmeßwert E₂ gemessen. Außerdem wurde während der gesamten Zeit der zeitabhängige Verlauf des Meßsignals bestimmt.

Die Bestimmung erfolgte an einem Boehringer Mannheim/Hitachi 717-Analysengerät unter Verwendung der Meßwellenlängenkombinationen 700/600 nm (Stand der Technik) sowie 505/570 nm bzw. 546/570 nm (Erfindung).

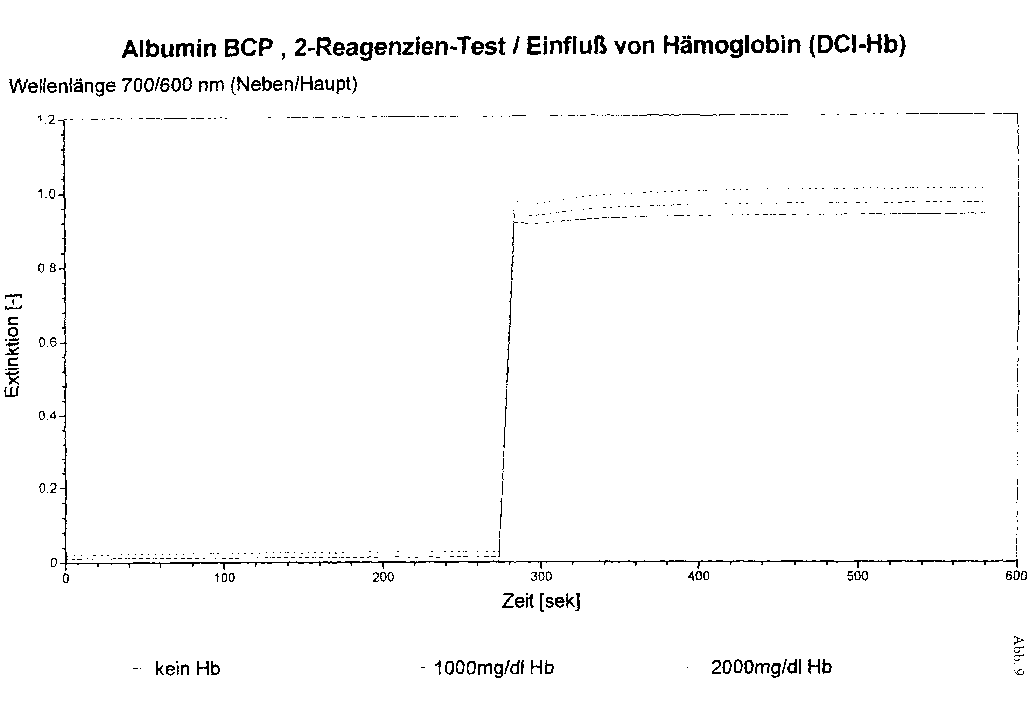

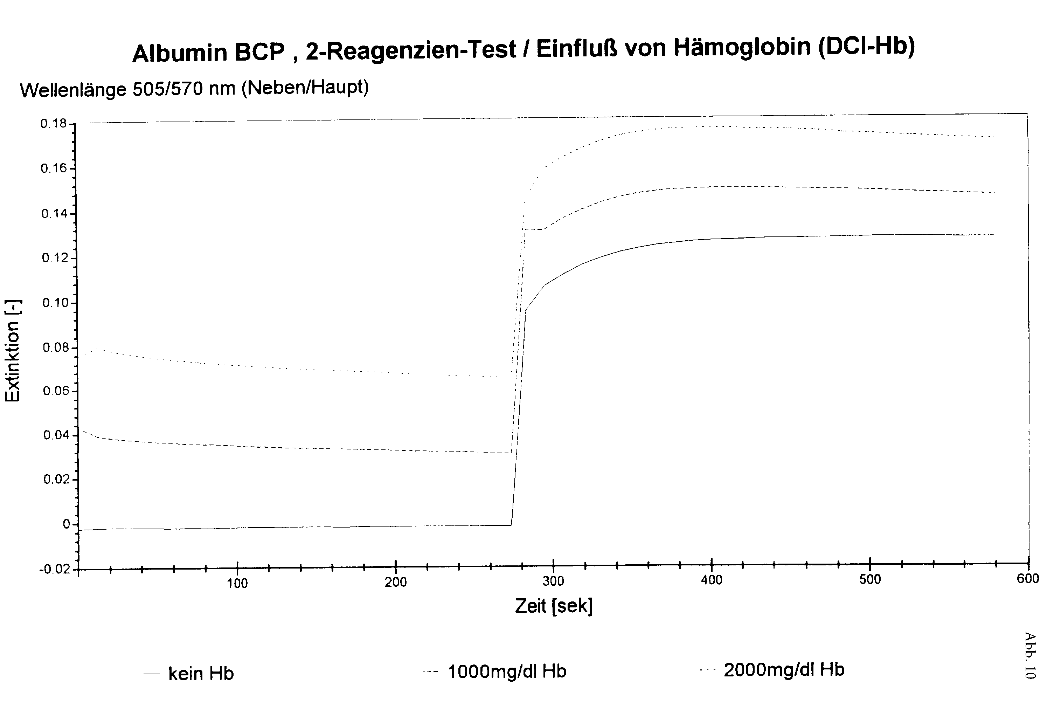

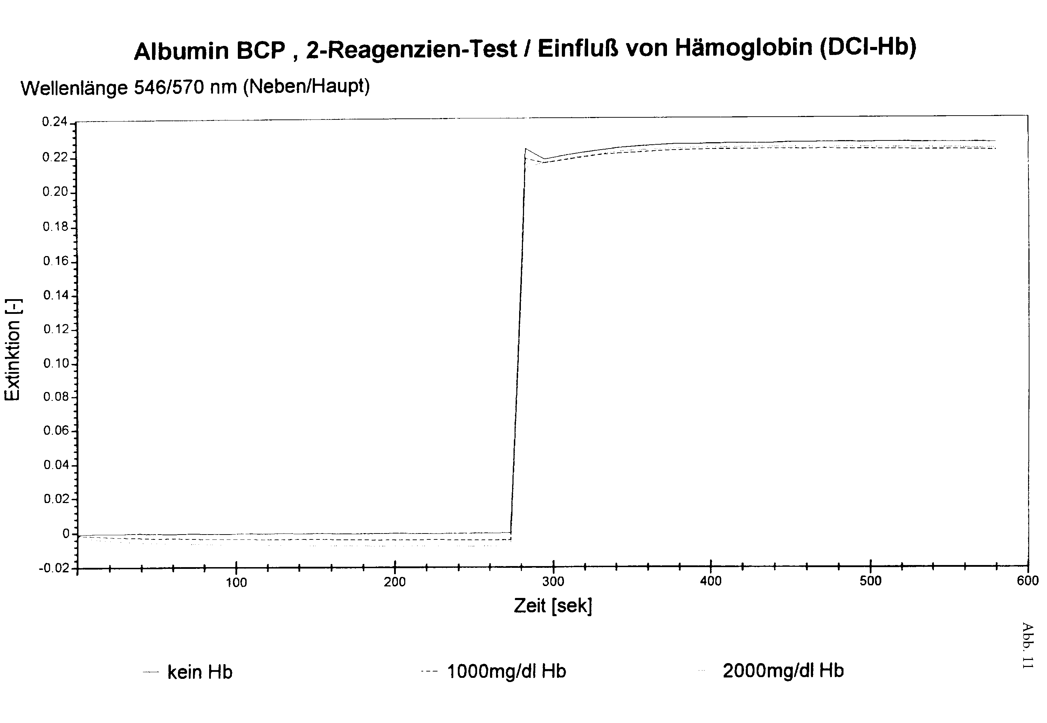

Der zeitabhängige Verlauf des Meßsignals für Proben ohne Hämoglobin, mit 1000 mg/dl Hämoglobin und mit 2000 mg/dl Hämoglobin in Form von DCL-Hämoglobin ist für die Wellenlängenkombination 700/600 nm in Abbildung 9 für die Wellenlängenkombination 505/570 nm in Abbildung 10 und für die Wellenlängenkombination 546/570 in Abbildung 11 dargestellt.

Es ist zu erkennen, daß bei der Wellenlängenkombination 700/600 nm der durch Hämoglobin verursachte Signalanstieg nach Zugabe von Reagenz 2 höher ist als vor Zugabe von Reagenz 2. Das ist ein Hinweis darauf, daß Hämoglobin selbst an der Reaktion mit Bromcresolpurpur teilnimmt und dadurch die Albuminbestimmung gestört wird. Das Meßsignal der hämoglobinhaltigen Proben wie auch der Probe ohne Hämoglobin ist jedoch vor Zugabe von Reagenz 2 wie auch nach Zugabe von Reagenz 2 nahezu konstant.

Demgegenüber ist bei der erfindungsgemäßen Wellenlängenkombination 505/570 nm mit zunehmender Reaktionsdauer eine Abnahme des Meßsignals bei den hämoglobinhaltigen Proben zu erkennen, während das Signal für die Probe ohne Hämoglobin im wesentlichen konstant bleibt. Zur Vermeidung von Störungen durch Hämoglobin sind die bevorzugten Reaktionszeitpunkte deshalb so zu wählen, daß unter Berücksichtigung des Verdünnungsfaktors das durch Hämoglobin verursachte Störsignal vor Zugabe von Reagenz 2 gleich oder annähernd gleich groß ist, wie das durch Hämoglobin verursachte Störsignal nach Zugabe von Reagenz 2. Im Beispiel 4 wird deshalb E₁, ca. 2,5 min (505/570 nm) bzw. 0,5 min (546/570 nm) nach Zugabe von Reagenz 1 zur Probe und E₂ bei beiden Wellenlängenkombinationen ca. 5 min nach Zugabe von Reagenz 2 gemessen.

In Tabelle 4 ist die prozentuale Wiederfindung des Albumingehaltes in Proben mit unterschiedlichem Hämoglobingehalt gezeigt. Bei den erfindungsgemäßen Meßwellenlängenkombinationen 505/570 nm und 546/570 nm wurde eine vom Hämoglobingehalt der Probe unabhängige Wiederfindung von 100 ± 4 % erreicht, während bei der Meßwellenlängenkombination 700/600 nm gemäß dem Stand der Technik mit zunehmendem Hämoglobingehalt eine starke Meßwertverfälschung festgestellt wurde.

Bei Proben, die Hämoglogin aus Hämolyse oder rekombinant hergestelltes Hämoglobin enthalten, konnte unter Verwendung der erfindungsgemäßen Wellenlängenkombinationen ebenfalls eine gute Entstörung erreicht werden.

### Beispiel 5

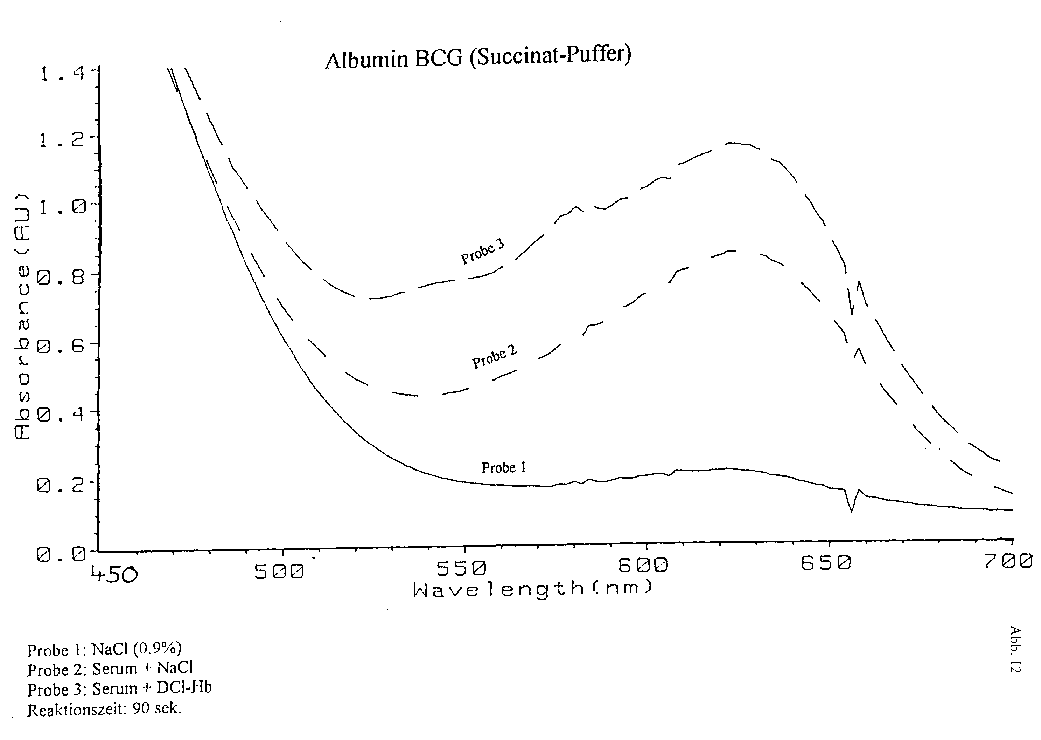

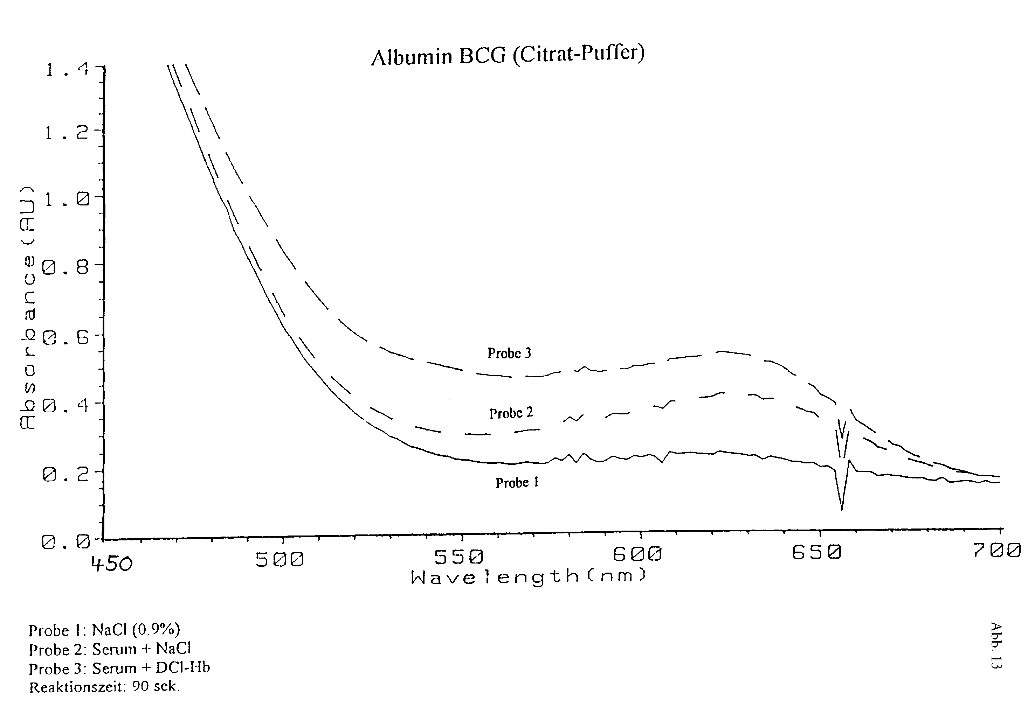

Die Abbildungen 12 und 13 zeigen wellenlängenabhängige Diodenarray-Spektren der Bestimmung von Albumin nach der Bromcresolgrün-Methode mit einem Monoreagenz in einem Succinat- bzw. Citratpuffer. Probe 1 ist eine Kontrolle mit 0,9 % NaCl. Probe 2 ist Serum + NaCl und Probe 2 ist Serum + DCL-Hämoglobin. Die Reaktionszeit war jeweils 90 s.

Aus Abb. 12 ist ersichtlich, daß neben den in Beispiel 1 angegebenen bevorzugten Wellenlängenkombinationen 546/570 bzw. 480/660 nm noch eine weitere Wellenlängenkombination 600/630 nm geeignet ist.

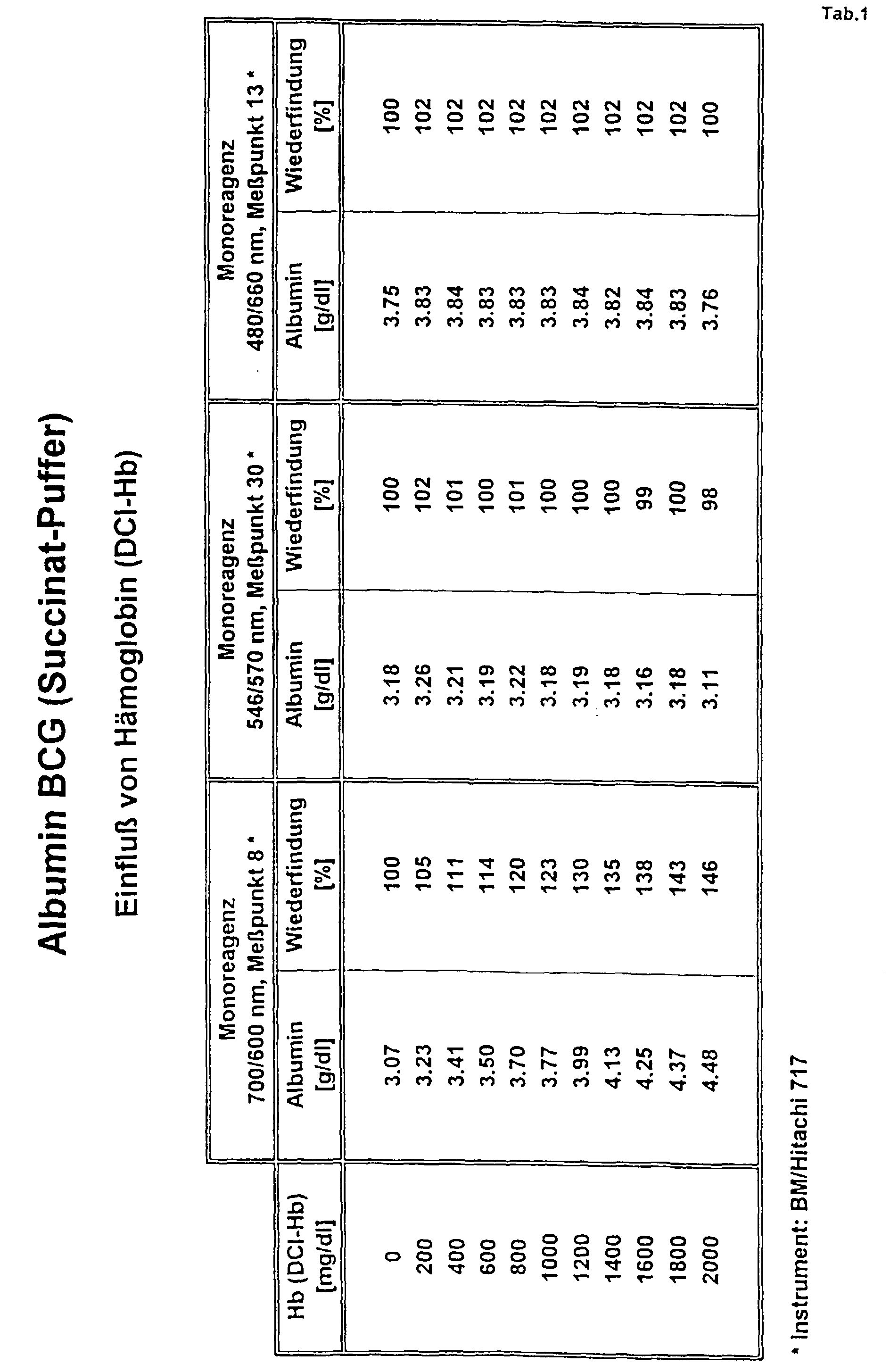

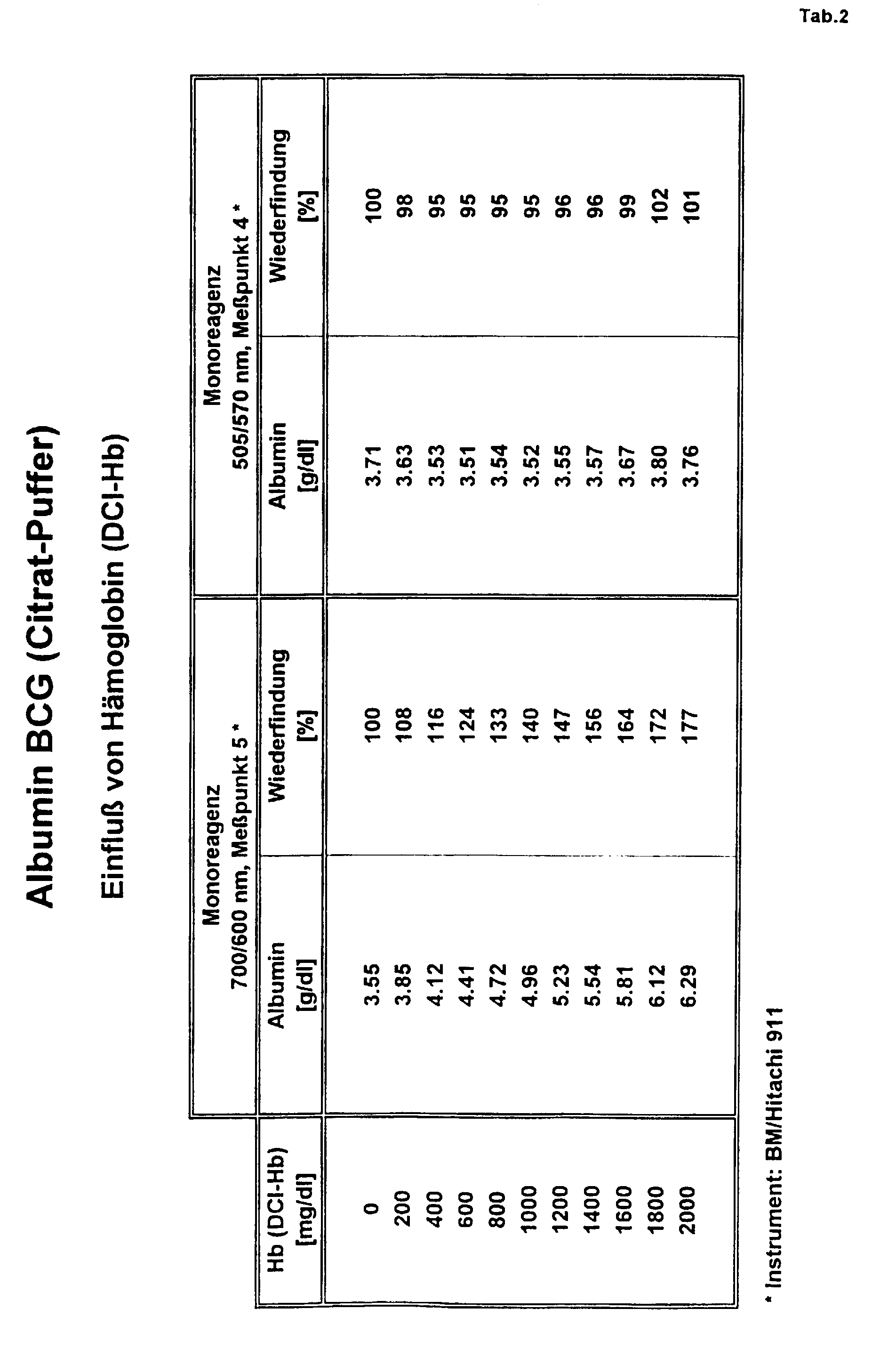

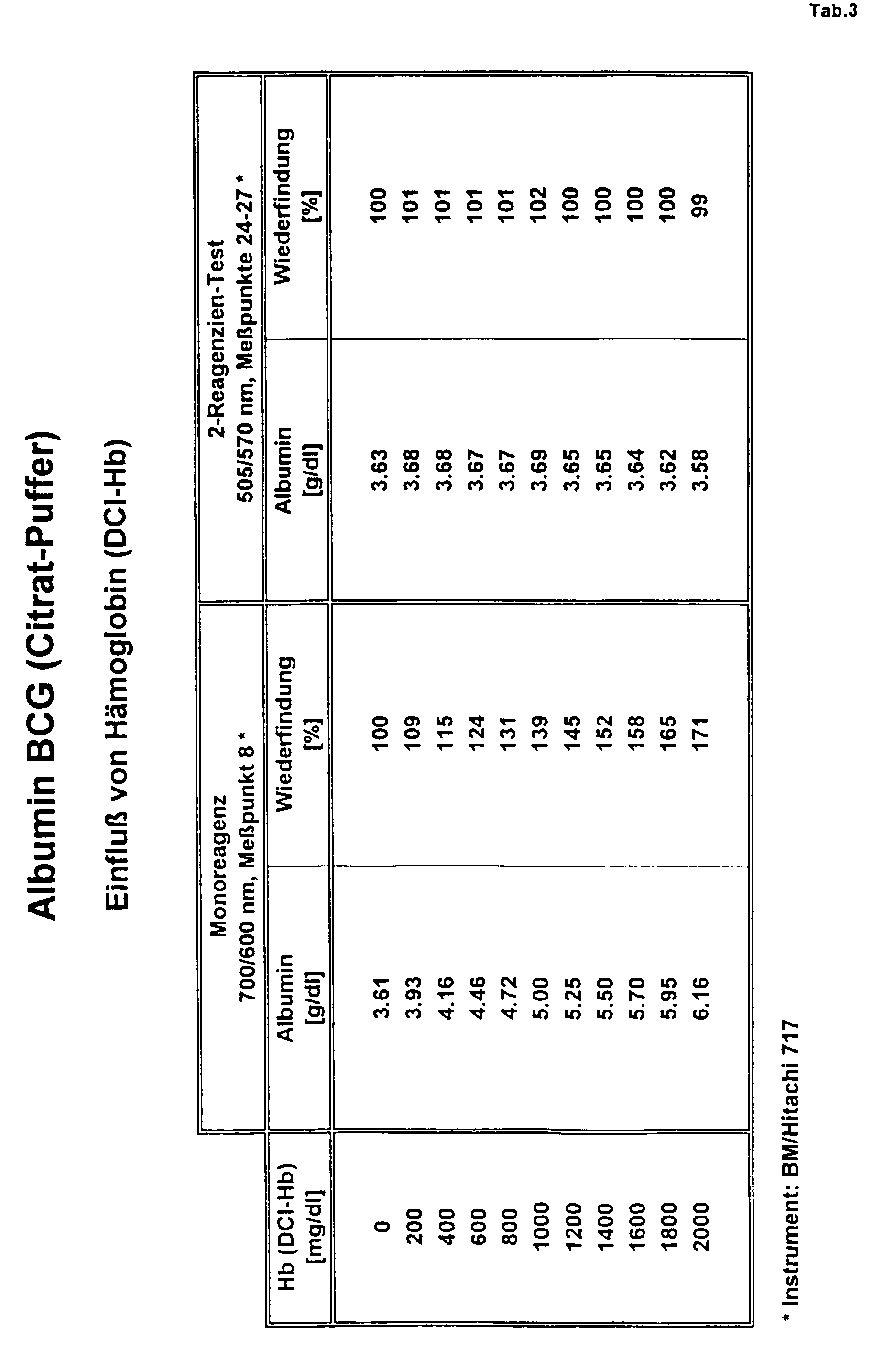

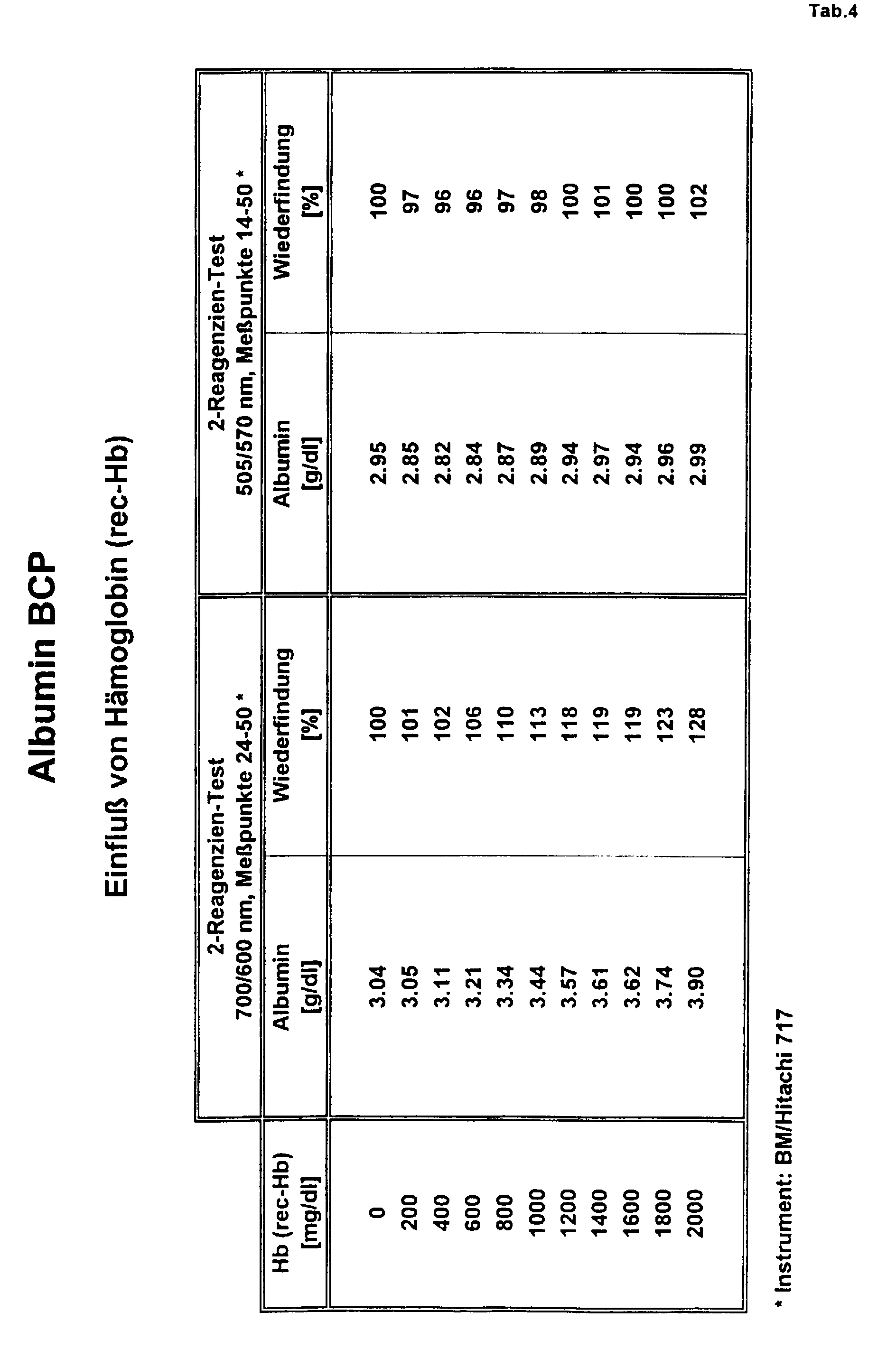

## Patentansprüche

1. Verfahren zur Bestimmung von Albumin in einer freies Hämoglobin enthaltenden Probe durch optische Messung bei mindestens zwei Wellenlängen,
**dadurch gekennzeichnet,** daß
(a) bei einer ersten Meßwellenlänge ein zur Bestimmung ausreichend hohes Meßsignal für Albumin vorliegt,
(b) bei einer zweiten Meßwellenlänge (i) kein Meßsignal für Albumin oder (ii) eine im Vergleich zum Meßsignal bei der ersten Meßwellenlänge geringeres Meßsignal für Albumin vorliegt und
(c) bei der ersten und bei der zweiten Meßwellenlänge ein vergleichbar hohes Störsignal vorliegt, das durch eine Reaktion von Hämoglobin mit dem zur Bestimmung von Albumin verwendeten Testreagenz erzeugt wird und sich zeitabhängig ändert.

2. Verfahren nach Anspruch 1,
**dadurch gekennzeichnet,** daß die Bestimmung als Einpunktmessung durchgeführt wird.

3. Verfahren nach Anspruch 1,
**dadurch gekennzeichnet,** daß die Bestimmung als Zwei- oder Mehrpunktmessung durchgeführt wird.

4. Verfahren nach einem der Ansprüche 1 bis 3,
**dadurch gekennzeichnet,** daß man die Bestimmung von Albumin mit der Bromcesolgrün-Methode in einem Succinat-Puffer durchführt.

5. Verfahren nach Anspruch 4,
**dadurch gekennzeichnet,** daß man die Bestimmung
(a) bei einer ersten Meßwellenlänge von 560 - 580 nm und bei einer zweiten Meßwellenlänge von 540 - 552 nm,
(b) bei einer ersten Meßwellenlänge von 640 - 680 nm und bei einer zweiten Meßwellenlänge von 470 - 490 nm oder
(c) bei einer ersten Meßwellenlänge von 620 - 640 nm und bei einer zweiten Meßwellenlänge von 590 - 610 nm durchführt.

6. Verfahren nach einem der Ansprüche 1 bis 3,
**dadurch gekennzeichnet,** daß man die Bestimmung von Albumin mit der Bromcresolgrün-Methode in einem Citrat-Puffer durchführt.

7. Verfahren nach Anspruch 6,
**dadurch gekennzeichnet,** daß man die Bestimmung bei einer ersten Meßwellenlänge von 560 bis 580 nm und bei einer zweiten Meßwellenlänge von 490 bis 520 nm durchführt.

8. Verfahren nach einem der Ansprüche 1 bis 3,
**dadurch gekennzeichnet,** daß man die Bestimmung von Albumin mit der Bromcresolpurpur-Methode durchführt.

9. Verfahren nach Anspruch 8,
**dadurch gekennzeichnet,** daß man die Bestimmung (a) bei einer ersten Meßwellenlänge von 560 bis 580 nm und bei einer zweiten Meßwellenlänge von 490 bis 520 nm oder (b) bei einer ersten Meßwellenlänge von 560 bis 580 nm und bei einer zweiten Meßwellenlänge von 540 bis 552 durchführt.

## Claims

1. Method for determining albumin in a sample containing free haemoglobin by optical measurement at at least two wavelengths
**wherein**
(a) the signal measured at a first measurement wavelength is sufficiently high to determine albumin,
(b) (i) no measured signal for albumin or (ii) a measured signal for albumin that is smaller than the measured signal at the first measurement wavelength, is present at a second measurement wavelength and
(c) an interfering signal of comparable magnitude is present at the first and at the second measurement wavelength which is caused by a reaction of haemoglobin with the test reagent used to determine albumin and which changes in a time-dependent manner.

2. Method as claimed in claim 1,
**wherein**
the determination is carried out as a one-point measurement.

3. Method as claimed in claim 1,
**wherein**
the determination is carried out as a two-point or multiple point measurement.

4. Method as claimed in one of the claims 1 to 3, **wherein**
albumin is determined using the bromocresol green method in a succinate buffer.

5. Method as claimed in claim 4,
**wherein**
the determination is carried out
(a) at a first measurement wavelength of 560 - 580 nm and at a second measurement wavelength of 540 - 552 nm,
(b) at a first measurement wavelength of 640 - 680 nm and at a second measurement wavelength of 470 - 490 nm or
(c) at a first measurement wavelength of 620 - 640 nm and at a second measurement wavelength of 590 - 610 nm.

6. Method as claimed in one of the claims 1 to 3,
**wherein**
albumin is determined using the bromocresol green method in a citrate buffer.

7. Method as claimed in claim 6,
**wherein**
the determination is carried out at a first measurement wavelength of 560 to 580 nm and at a second measurement wavelength of 490 to 520 nm.

8. Method as claimed in one of the claims 1 to 3,
**wherein**
albumin is determined using the bromocresol purple method.

9. Method as claimed in claim 8,
**wherein**
the determination is carried out (a) at a first measurement wavelength of 560 to 580 nm and at a second measurement wavelength of 490 to 520 nm or (b) at a first measurement wavelength of 560 to 580 nm and at a second measurement wavelength of 540 to 552 nm.

## Revendications

1. Procédé de dosage d'albumine dans un échantillon contenant de l'hémoglobine libre, par mesure optique à au moins deux longueurs d'onde, caractérisé en ce que
(a) à une première longueur d'onde de mesure, il y a un signal de mesure pour l'albumine suffisamment élevé pour le dosage,
(b) à une deuxième longueur d'onde de mesure, (i) il n'y a pas de signal de mesure pour l'albumine ou (ii) il y a un signal de mesure pour l'albumine plus faible par comparaison au signal de mesure à la première longueur d'onde de mesure, et
(c) à la première et à la deuxième longueur d'onde de mesure, il y a un signal de perturbation de hauteur comparable qui est produit par une réaction de l'hémoglobine avec le réactif d'analyse utilisé pour le dosage de l'albumine et qui varie en fonction du temps.

2. Procédé selon la revendication 1, caractérisé en ce que le dosage s'effectue sous forme d'une mesure à un point.

3. Procédé selon la revendication 1, caractérisé en ce que le dosage s'effectue sous forme d'une mesure à deux ou plusieurs points.

4. Procédé selon l'une des revendications 1 à 3, caractérisé en ce que l'on effectue le dosage de l'albumine par la méthode du vert de bromocrésol dans un tampon succinate.

5. Procédé selon la revendication 4, caractérisé en ce que l'on effectue le dosage
(a) à une première longueur d'onde de mesure de 560 à 580 nm et à une seconde longueur d'onde de mesure de 540 à 552 nm,
(b) à une première longueur d'onde de mesure de 640 à 680 nm et à une seconde longueur d'onde de mesure de 470 à 490 nm, ou
(c) à une première longueur d'onde de mesure de 620 à 640 nm et à une seconde longueur d'onde de mesure de 590 à 610 nm.

6. Procédé selon l'une des revendications 1 à 3, caractérisé en ce que l'on effectue le dosage de l'albumine par la méthode du vert de bromocrésol dans un tampon citrate.

7. Procédé selon la revendication 6, caractérisé en ce que l'on effectue le dosage à une première longueur d'onde de mesure de 560 à 580 nm et à une seconde longueur d'onde de mesure 490 à 520 nm.

8. Procédé selon l'une des revendications 1 à 3, caractérisé en ce que l'on effectue le dosage de l'albumine par la méthode du pourpre de bromocrésol.

9. Procédé selon la revendication 8, caractérisé en ce que l'on effectue le dosage
(a) à une première longueur d'onde de mesure de 560 à 580 nm et à une seconde longueur d'onde de mesure de 490 à 520 nm, ou
(b) à une première longueur d'onde de mesure de 560 à 580 nm et à une seconde longueur d'onde de mesure de 540 à 552 nm.
